(19) Europäisches Patentamt
European Patent Office
Office européen des brevets

(11) EP 4 190 907 A1

(12) EUROPEAN PATENT APPLICATION
published in accordance with Art. 153(4) EPC

(43) Date of publication:
07.06.2023 Bulletin 2023/23

(51) International Patent Classification (IPC):
C12P 7/52 (2006.01)    C12N 15/70 (2006.01)
C12N 9/04 (2006.01)    C12N 9/02 (2006.01)

(21) Application number: 21848600.9

(22) Date of filing: 30.07.2021

(52) Cooperative Patent Classification (CPC):
C12N 9/0004; C12N 15/70; C12P 7/52

(86) International application number:
PCT/KR2021/009988

(87) International publication number:
WO 2022/025710 (03.02.2022 Gazette 2022/05)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 31.07.2020 KR 20200096238

(71) Applicant: LG Chem, Ltd.
Seoul 07336 (KR)

(72) Inventors:
• KIM, Jae Hyung
Daejeon 34122 (KR)

• KANG, Donggyun
Daejeon 34122 (KR)
• LEE, Kyung Muk
Daejeon 34122 (KR)

(74) Representative: Goddar, Heinz J. et al
Boehmert & Boehmert
Anwaltspartnerschaft mbB
Pettenkoferstrasse 22
80336 München (DE)

Remarks:
The complete document including Reference
Table(s) and the Sequence Listing(s) can be
downloaded from the EPO website

(54) METHOD FOR PREPARING 3-HYDROXYPROPIONIC ACID THROUGH TWO STEPS

(57) The present invention relates to a method for preparing 3-hydroxypropionic acid (3-HP) and/or a method for improving the productivity of 3-HP, the methods comprising the steps of: (1) performing high-concentration cell culturing of a 3-HP-producing strain; and (2) isolating high-concentration-cultured cells to inoculate a medium for 3-HP production with same, thereby producing 3-HP, and thus the present invention can improve the productivity and yield of 3-HP.

[FIG. 2]

**Description**

[TECHNICAL FIELD]

The present invention relates to a method for manufacturing 3-hydroxypropionic acid (3-HP) and/or a method for improving productivity of 3-HP, and more specifically, relates to a two-step method for manufacturing comprising a first step of high concentration cell culture and a second step of producing 3-HP using the high concentration cells cultured as a catalyst.

Cross-reference to related applications

The present application claims the benefit of the priority based on Korean patent application No. 10-2020-0096238 on July 31, 2020, and the entire contents disclosed in the corresponding Korean patent application are incorporated herein as a part.

[BACKGROUND ART]

3-hydroxypropionic acid is a platform compound that can be converted into various chemical substances such as acrylic acid, methyl acrylate, acrylamide, and the like. Since it was selected as one of the Top 12 value-added bio-chemicals by the US Department of Energy (DOE) in 2004, it has been actively studied in academia and industry.
The production of 3-HP is largely made up of two methods of a chemical method and a biological method, but in the case of the chemical method, it is pointed out that it is not eco-friendly as the initial material is expensive and toxic substances are generated during the production process, so an eco-friendly bio-process is in the spotlight.
Glucose and glycerol are mainly used as a substrate for 3-HP biosynthesis using a microorganism, and currently, research on a one-step fermentation method in which 3-HP production and cell growth occur simultaneously is mainly being conducted.
However, during the first step production as described above, the substrate for producing 3-HP is also used for cell growth, and thus by-products such as acetate or lactate are generated, and thereby, there is a problem that the yield and productivity of 3-HP are degraded. Due to such low yield and productivity, despite the possibility of 3-HP, commercialization has not yet been reached.

[BRIEF SUMMARY OF INVENTION]

[TECHNICAL PROBLEM]

One embodiment of the present application is to provide a method for manufacturing of 3-hydroxypropionic acid (3-HP), comprising (1) performing high concentration cell culturing of a 3-hydroxypropionic acid (3-HP) producing strain; and (2) producing 3-hydroxypropionic acid by separating the cells cultured at a high concentration and inoculating and/or culturing them to a medium for producing 3-hydroxypropionic acid, and/or a method for improving the productivity of 3-HP.
Through the two-step method for manufacturing, the degradation of the 3-HP productivity due to cell growth or generation of by-products may be improved.
Other example provides a culture solution of a 3-hydroxypropionic acid producing strain comprising 3-hydroxypropionic acid at a high concentration. In one example, the culture solution may be produced by the two-step method for manufacturing described above. The culture solution may be used for use in production of 3-hydroxypropionic acid.

[TECHNICAL SOLUTION]

One example,
provides a method for manufacturing of 3-hydroxypropionic acid (3-HP) and/or a method for improving the productivity of 3-HP, comprising

> (1) high concentration cell culturing a 3-hydroxypropionic acid (3-HP) producing strain; and
> (2) producing 3-hydroxypropionic acid by separating the cells cultured at a high concentration and inoculating and/or culturing them to a medium for producing 3-hydroxypropionic acid.

Another example provides a culture solution of a 3-hydroxypropionic acid producing strain comprising 3-hydroxypropionic acid at a high concentration. The culture solution may be used for use in production of 3-hydroxypropionic acid. The culture solution may be produced by the method for manufacturing.

Hereinafter, the present invention will be described in more detail.

2-step 3-HP production

(1) High concentration cell culture step of 3-HP producing strain

The method for manufacturing of 3-HP and/or method for improving the productivity of 3-HP provided herein comprises (1) high concentration cell culturing of a 3-hydroxypropionic acid producing strain.

The 3-hydroxypropionic acid producing strain may be selected among all natural or genetically modified recombinant microorganisms capable of producing 3-hydroxypropionic acid. In one example, the microorganism may be selected from microorganisms consisting of Escherichia sp. (for example, E. coli, etc.), Pseudomonas sp., Enterobacteria sp., Brevibacterium sp., Corynebacterium sp., Klebsiella sp., Citrobacter sp., Clostridium sp., Streptomyces sp., Bacillus sp., Lactobacillus sp., Pseudomonas sp., Saccharomyces sp. and Aspergillus sp., but not limited thereto. In one specific example, the 3-hydroxypropionic acid producing strain may be E. coli.

The 3-hydroxypropionic acid producing strain may comprise a gene encoding one or more or two kinds of the proteins selected from the group consisting of glycerol dehydratase and aldehyde dehydrogenase. In one example, the 3-HP producing strain may further comprise a gene (gdrAB) encoding glycerol dehydratase reactivase (GdrAB). In one example, the 3-HP producing strain may be a strain which can further biosynthesize vitamin $B_{12}$.

The glycerol dehydratase may be encoded by the dhaB (GenBank accession no. U30903.1) gene, but not limited thereto. The dhaB gene may be an enzyme derived from *Klebsiella pneumonia*, but not limited thereto. The gene encoding glycerol dehydratase may comprise a gene encoding dhaB1, dhaB2 and/or dhaB3. The glycerol dehydratase protein and gene encoding it may comprise a mutation of the gene and/or amino acid sequence within a range that maintains the activity of the enzyme which degrades glycerol into 3-hydroxypropanal (3-HPA) and water ($H_2O$).

The gene (aldH) encoding aldehyde dehydrogenase (ALDH) may be for example, aldH (GenBank Accession no. U00096.3; EaldH) gene derived from Escherichia coli or *E. coli* K12 MG1655 cell line, puuC gene derived from *K. pneumonia* and/or KGSADH gene derived from *Azospirillum brasilense*, but not limited thereto. The aldehyde dehydrogenase protein and gene encoding it may comprise a mutation of the gene and/or amino acid sequence within a range that maintains the activity to produce 3-HP from 3-HPA.

The gene encoding glycerol dehydratase reactivase (GdrAB) may be gdrAB gene derived from *Klebsiella pneumonia* (*K. pneumonia*).

The 3-HP producing strain may comprise a recombinant vector comprising a gene encoding one or more, two or more or all 3 kinds of proteins selected from the group consisting of glycerol dehydratase, aldehyde dehydrogenase and glycerol dehydratase reactivase.

The recombinant vector may be used by replacing a promoter or regulation site in a range of purpose to express a gene encoding one or more, two or more or all 3 kinds of proteins selected from the group consisting of glycerol dehydratase, aldehyde dehydrogenase and glycerol dehydratase reactivase within a cell by a method known in the art.

The high concentration culture may be performed using a method known in the art in a range of purpose to secure a large amount of 3-HP producing strain without limitation, and in one example, the culture may be performed by fed-batch culture.

In one example, the fed-batch culture may be performed by pH-stat method, continuous feeding culture method or combination thereof. In one embodiment, when the fed-batch culture of pH-stat method is performed, glucose may be added in a concentration of 1 to 5g/L, but not limited thereto. In one embodiment, when the fed-batch culture of continuous feeding culture is performed, glucose may be injected at a rate of 10 to 20g/L/h, but not limited thereto.

In one example, during the high concentration culture, pH may be maintained at 5 to 7.5, 5 to 7, 5.5 to 7.5, 5.5 to 7, 6 to 7.5 or 6.5 to 6, but not limited thereto.

In one example, during the high concentration culture, as a carbon source, a monosaccharide, a disaccharide and/or a polysaccharide may be selected and used in a range of purpose without limitation. For example, the carbon source may be one or more, two or more, three or more, four or more, five or more, ten or more, or a combination of all 11 kinds selected from the group consisting of glucose, fructose, galactose, mannose, arabinose, xylose, ribose, sucrose, maltose, lactose, and cellobiose. The medium used during the high concentration culture may not comprise glycerol as a carbon source. As such, since the medium used in the high concentration culture does not comprise glycerol, 3-HP production may not occur in the high concentration step.

In one example, after the high concentration cell culture, the cell concentration may be 10 or more, 30 or more, 50 or more, 70 or more, 100 or more, or 110 or more, based on the $OD_{600}$ value, and for example, may be 10 or more, 50 or more, 100 or more, 150 or more, 200 or more, and in case of cell culture for 20 hours, the $OD_{600}$ value may be 10 to 500, 10 to 400, 10 to 300, 10 to 250, 10 to 200, 10 to 150, 30 to 500, 30 to 400, 30 to 300, 30 to 250, 30 to 200, 30 to 150, 50 to 500, 50 to 400, 50 to 300, 50 to 250, 50 to 200, 50 to 150, 70 to 500, 70 to 400, 70 to 300, 70 to 250, 70 to 200, 70 to 150, 100 to 500, 100 to 400, 100 to 300, 100 to 250, 100 to 200, 100 to 150, 110 to 500, 110 to 400, 110 to 300, 110 to 250, 110 to 200, or 110 to 150, and for example, may be 120, but not limited thereto.

In one example, after the high concentration cell culture, the cell concentration may be 10 to 100g/L, 10 to 80g/L, 10 to 50g/L, 10 to 40g/L, 10 to 35g/L, 15 to 100g/L, 15 to 80g/L, 15 to 50g/L, 15 to 40g/L, 15 to 35g/L, 20 to 100g/L, 20 to 80g/L, 20 to 50g/L, 20 to 40g/L, 20 to 35g/L, 25 to 100g/L, 25 to 80g/L, 25 to 50g/L, 25 to 40g/L, or 25 to 35g/L, based on cell dry weight (g) per 1L of medium, but not limited thereto.

(2) 3-HP producing step

The manufacturing method of 3-HP and/or method for improving the productivity of 3-HP provided herein comprises (2) producing 3-hydroxypropionic acid by separating the cells cultured at a high concentration and inoculating and/or culturing them in a medium for producing 3-hydroxypropionic acid.

The separation of the cells cultured at a high concentration may be performed by one or more, two or more or all of the steps consisting of centrifuging cells, removing supernatant and resuspending pellets with buffer. In one example, the buffer may be PBS (Phosphate buffered saline), but not limited thereto.

The medium for producing 3-hydroxypropionic acid may be used without limitation in a range of purpose to allow the strain to produce 3-HP without further growth of the 3-HP producing strain.

In one example, the carbon source of the medium for producing 3-hydroxypropionic acid may be glycerol, but not limited thereto. In one example, the medium for producing may further comprise vitamin $B_{12}$. The medium for producing 3-hydroxypropionic acid may not comprise glucose as a carbon source.

In one example, the medium may be synthetic media or semisynthetic media, but not limited thereto.

In the inoculation of the cells cultured at a high concentration, in a range of purpose for 3-HP production, those skilled in the art may appropriately change the cell inoculation concentration. In one example, the cell concentration during inoculation (based on dry cell weight (DCW) per 1L of medium) may be 1 to 20 g/L, 1 to 16 g/L, 1 to 12 g/L, 1 to 9 g/L, 2 to 20g/L, 2 to 16 g/L, 2 to 12g/L, 2 to 9 g/L, 4 to 20 g/L, 4 to 16 g/L, 4 to 12 g/L, or 4 to 9 g/L, but not limited thereto.

The producing 3-hydroxypropionic acid may select and use a culture method known in the art in a range of purpose to produce 3-HP without limitation, and in one example, the culture may be performed by fermentation culture.

In the producing 3-HP, the proliferation of the inoculated cells may not occur. In one example, at the end of the producing, the number of cells may be 150% or less, 130% or less, 100% or less, 90% or less, 80% or less, 50 to 150%, 50 to 130%, 50 to 100%, 50 to 90%, 50 to 80%, 70 to 150%, 70 to 130%, 70 to 100%, 70 to 90%, or 70 to 80% of the number of the inoculated cells at the start of the producing, but not limited thereto.

The 3-HP yield of the method for manufacturing of 3-HP and/or method for improving the productivity of 3-HP provided herein, may be for example, based on culture (3-HP production) for 29 hours, 80% or more, 85% or more, 90% or more, 93% or more, or 95% or more, but not limited thereto. The 3-HP yield may be calculated as the 3-HP production amount in the medium compared to the amount of glycerol used (number of moles) in the medium in the step (2), and in one example, it may be calculated as Equation 1 below.

[Equation 1]

$$\text{Yield (\%)} = (\text{Final 3-HP (g)})/\{(\text{Glycerol before fermentation (g)}) - (\text{remaining glycerol after fermentation) (g)}\}$$

The 3-HP productivity (amount of production of 3-HP per 1L of medium for 1 hour (g)) of the method for manufacturing of 3-HP and/or method for improving the productivity of 3-HP provided herein may be 2.0 g/L/h or more, 2.5 g/L/h or more, 2.0 to 30 g/L/h, 2.0 to 20 g/L/h, 2.0 to 10 g/L/h, 2.0 to 5 g/L/h, 2.5 to 30 g/L/h, 2.5 to 20 g/L/h, 2.5 to 10 g/L/h, or 2.5 to 5 g/L/h, but not limited thereto.

In one embodiment, as the result of manufacturing 3-HP by the two-step method for manufacturing 3-HP of the present invention, the productivity was measured as 2.94g/L/h, and this is the highest among values reported in documents to date.

The amount of 3-HP production of the method for manufacturing of 3-HP and/or method for improving the productivity of 3-HP provided herein, may be, for example, 40g/L or more, 41g/L or more, 42g/L or more, 43g/L or more, 44g/L or more, 45g/L or more, 46g/L or more, 47g/L or more, 48g/L, 49g/L or more, 50g/L or more, 51g/L or more, 52g/L or more, or 53g/L or more, based on the 3-HP content (g) per 1L of medium during 20 to 30 hours (for example, 20 hours, 21 hours, 22 hours, 23 hours, 24 hours, 25 hours, 26 hours, 27 hours, 28 hours, 29 hours, or 30 hours) culture (3-HP production) (the upper limit may be selected from 60 to 1000g/L without particular limitation, and for example, may be 1000g/L, 500g/L, 100g/L, 90g/L, 80g/L, 70g/L, or 60g/L, but not limited thereto).

The method for manufacturing of 3-HP and/or method for improving the productivity of 3-HP provided herein may not produce by-products according to 3-HP production. The by-products may be acetate and/or lactate. In one example, the by-products may be produced as the concentration in the total culture solution is 1%(w/v) or less, 0.5%(w/v) or less, 0.1%(w/v) or less, 0.01%(w/v) or less, 0.001%(w/v) or less, 0.0001%(w/v), or 0.00001%(w/v) or less (then, the lower limit of the concentration of by-products may be selected from 0 to 0.000001(w/v), but not limited thereto), or may be produced at a concentration of 0%(w/v) (no by-products are produced in a detectable concentration).

The two-step method for manufacturing 3-HP provided herein, may have the 3-HP yield (%) 1.05 times or more or 1.1 times or more higher, for example, 1.05 to 10 times or more, 1.05 to 5 times or more, 1.05 to 2 times or more, 1.05 to 1.7 times or more, 1.05 to 1.5 times or more, 1.05 to 1.2 times or more, 1.1 to 10 times or more, 1.1 to 5 times or more, 1.1 to 2 times or more, 1.1 to 1.7 times or more, 1.1 to 1.5 times or more or 1.1 to 1.2 times or more higher, for example, about 1.13 times higher, than the one-step fermentation method in which cell growth and 3-HP production occur simultaneously.

The two-step method for manufacturing 3-HP provided herein may have the 3-HP productivity (g/L/h) of 1.1 times or more, 1.3 times or more, or 1.5 times or more higher, than the one-step fermentation method, and for example, it may be 1.1 to 10 times, 1.1 to 5 times, 1.1 to 3 times, 1.1 to 2.5 times, 1.1 to 2 times, 1.3 to 10 times, 1.3 to 5 times, 1.3 to 3 times, 1.3 to 2.5 times, 1.3 to 2 times, 1.5 to 10 times, 1.5 to 5 times, 1.5 to 3 times, 1.5 to 2.5 times, 1.5 to 2 times, 1.9 to 3 times, 1.9 to 2.5 times or 1.9 to 2 times higher, and for example, it may be about 1.96 times higher.

The two-step method for manufacturing 3-HP provided herein may further comprise separating, collecting and/or purifying 3-hydroxypropionic acid from the culture solution, after (2) producing 3-hydroxypropionic acid by separating the cells cultured at a high concentration and inoculating and/or culturing them in a medium for producing 3-hydroxypropionic acid.

Culture solution comprising 3-HP

Other example of the present application provides a culture solution of a 3-hydroxypropionic acid producing strain comprising 3-hydroxypropionic acid at a high concentration. The culture solution may not comprise or comprise cells (3-hydroxypropionic acid producing strain). The culture solution may not comprise glucose.

The 3-hydroxypropionic acid producing strain is same as described above.

The culture solution may have 3-hydroxypropionic acid of 41g/L or more, 42g/L or more, 43g/L or more, 44g/L or more, 45g/L or more, 46g/L or more, 47g/L or more, 48g/L, 49g/L or more, 50g/L or more, 51g/L or more, 52g/L or more, or 53g/L or more (the upper limit may be selected from 60 to 1000g/L without particular limitation, and for example, it may be 1000g/L, 500g/L, 100g/L, 90g/L, 80g/L, 70g/L, or 60g/L, but not limited thereto). In the culture solution, the concentration of by-products selected from the group consisting of acetate and lactate may be 1%(w/v) or less, 0.5%(w/v) or less, 0.1%(w/v) or less, 0.01%(w/v) or less, 0.001%(w/v), 0.0001%(w/v), or 0.00001%(w/v) or less (then, the lower limit of the concentration of by-products may be selected from 0 to 0.000001(w/v), but not limited thereto), or may be 0%(w/v).

The culture solution may be obtained by the aforementioned step (1) and step (2).

The culture solution may be used for use in production of 3-hydroxypropionic acid.

Other example provides a composition for producing 3-hydroxypropionic acid comprising the culture solution.

Other example provides a method for producing 3-hydroxypropionic acid comprising separating, collecting and/or purifying 3-hydroxypropionic acid from the composition for producing 3-hydroxypropionic acid.

[ADVANTAGEOUS EFFECTS]

The two-step method for manufacturing 3-HP provided by the present invention may be usefully used for commercialization of 3-HP, as the productivity and yield of 3-HP are significantly improved, compared to one-step culture.

[BRIEF DESCRIPTION OF THE DRAWINGS]

FIG. 1 is a graph showing the OD change according to the change of time as the result of performing high concentration cell culture of a 3-HP producing strain according to one example of the present invention.

FIG. 2 is a graph showing the result of measuring the concentration of 3-HP (3HP), glycerol, acetate and lactate over time, after inoculating the 3-HP producing strain cultured at a high concentration according to one example of the present invention in a medium for producing 3-HP (0h).

[MODE FOR INVENTION]

Hereinafter, the present invention will be described in more detail by examples. However, the following examples are intended to illustrate the contents of the present invention only, but the scope of the present invention is not limited by the following examples.

Unless otherwise mentioned herein, all temperatures are based on degrees Celsius, and nucleic acid sequences are described from the 5' end to the 3' end unless there is a particular circumstance.

Example 1. High concentration cell culture of 3-HP producing strain

1-1. Manufacturing of 3-HP producing strain

A recombinant vector in which a gene encoding glycerol dehydratase and aldehyde dehydrogenase known to produce 3-hydroxypropionic acid (3-HP) using glycerol as a substrate is introduced was manufactured. A 3-HP producing strain was produced by introducing the manufactured recombinant vector into E. coli W3110 strain.

Specifically, a recombinant vector for producing 3HP (pCDF_J23101_dhaB_gdrAB_J23100_aldH) was produced by cloning a gene encoding glycerol dehydratase (dhaB), a gene encoding aldehyde dehydrogenase (aldH) and a gene encoding glycerol dehydratase reactivase (gdrAB) in a plasmid pCDF.

The pCDFDuetJ23 vector used for production of the recombinant vector is a vector in which the promoter portion of the pCDFDuet-1 vector was substituted with J23101 and J23100 promoters. The dhaB (U30903.1; about 2.7 kb; comprising dhaB1, dhaB2 and dhaB3) and gdrAB gene (about 2.2 kb; gdrA, gdrB) inserted into the vector were amplified using the primers of Table 1 below in the chromosome of Klebsiella pneumonia (ATCC 25955). Since the dhaB123 and gdrA genes were located side by side on the chromosome of Klebsiella pneumonia, they were amplified together, and as gdrB was located in the opposite direction to dhaB123 and gdrA, only gdrB was amplified separately.

The aldH gene was amplified and isolated from the genome of E. coli K12 MG1655 strain using the promoter pair consisting of the nucleic acid sequences of SEQ ID NO: 5 and SEQ ID NO: 6 of Table 1 below. In Table 1 below, the nucleic acid sequences of primers used for amplification of each gene were shown, and among names, '-F' means a forward promoter, and '-R-'means a reverse promoter.

[Table 1]

| SEQ ID NO: | Name | Nucleic acid sequence (5'>3') |
|---|---|---|
| 1 | dhaB-gdrA-F | GAATTCATGAAAAGATCAAAACGATTTGCAGTCCT |
| 2 | dhaB-gdrA-R | AAGCTTGATCTCCCACTGACCAAAGCTGG |
| 3 | gdrB-F | AAGCTTAGAGGGGGCCGTCATGTCGCTTTCACCGCCA G |
| 4 | gdrB-R | CTTAAGTCAGTTTCTCTCACTTAACGGC |
| 5 | aldH-F | ggtaccatgaattttcatcatctggc |
| 6 | aldH-R | catatgtcaggcctccaggcttat |

After amplification of each gene, dhaB123 and gdrA genes using restriction enzymes EcoRI and HindIII, and gdrB gene using restriction enzymes HindIII and AflII were cloned in the downstream of J23101 promoter of the pCDFDuetaJ23 vector. aldH was cloned on the bottom of J23108 promoter using restriction enzymes KpnI and NdeI. The cloning method of each gene was performed by a method known in the art.

The plasmid was introduced into E. coli W3110 (KCCM 40219) by electroporation, to prepare a 3HP producing strain.

**1-2. High concentration culture of cells**

The prepared 3-HP producing strain was subjected to high concentration cell culture in a 5L fermenter (Working volume 2L) using the fed-batch culture method.

Specifically, MR medium ($KH_2PO_4$ 6.67g, $(NH_4)_2HPO_4$ 4g, MgSO4 · $7H_2O$ 0.8g, citric acid 0.8g, and trace metal solution 5mL per 1 L; herein, Trace metal solution is 5M HCl 5mL, $FeSO_4$ · $7H_2O$ 10g, $CaCl_2$ 2g, $ZnSO_4$ · $7H_2O$ 2.2g, $MnSO_4$ · $4H_2O$ 0.5g, $CuSO_4$ · $5H_2O$ 1g, $(NH_4)_6Mo_7O_2$ · $4H_2O$ 0.1g, and $Na_2B_4O_2$ · $10H_2O$ 0.02g per 1 L) was used as a cell culture medium by adding glucose 20g/L and antibiotics (streptomycin) 25mg/L, and the temperature of 35 degrees Celsius was maintained. pH was maintained at 6.95 using ammonia water, and the amount of dissolved oxygen (DO) was maintained at 20% while increasing the stirring rate stepwise up to 900rpm, and the aeration was maintained at 1vvm.

For the fed-batch culture, the pH-stat feeding method was used, and glucose was added at 3g/L.

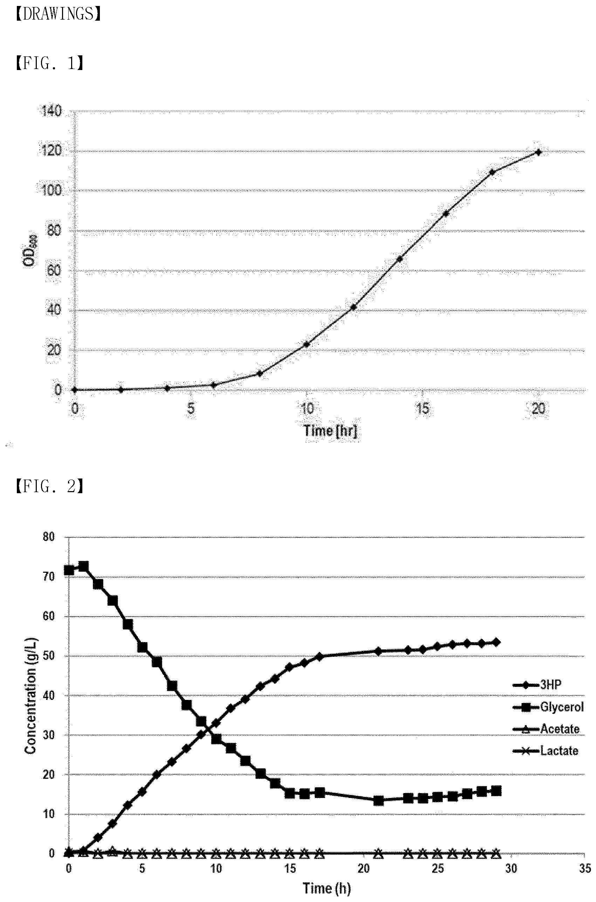

As the culture time elapsed, the cell concentration was measured by measuring the absorbance (Optical Density, OD) using a UV-Spectrometer. In FIG. 1, a graph of changes of $OD_{600}$ values over time was shown. At 20 hours after the start of the culture, $OD_{600}$ was shown as about 120 (dry cell weight 30g/L).

After completing the high concentration cell culture for 20 hours, the cells were collected by centrifuging the cell culture solution at 6,000rpm at 4 degrees Celsius for 10 minutes. The collected cells were suspended with PBS (phosphate-buffered saline) and used for the following step.

**Example 2. 3-HP production step**

A medium for producing 3-HP was prepared by adding glycerol 70g/L and vitamin $B_{12}$ 50uM to M9 medium without glucose. The cell suspension prepared in Example 1-2 was inoculated to the medium for producing 3-HP so that the cell inoculum amount was 5g/L (based on dry cell weight), and the 3-HP production step was performed in a 5L fermenter (Working volume 2L).

The culture condition for 3-HP production was maintained as the temperature of 35°C Celsius, the stirring rate of 300rpm and the aeration of 1 vvm, and the pH was maintained at 7.0 using $Ca(OH)_2$.

The concentration of 3-HP, glycerol, acetate and lactate was measured by high pressure lipid chromatography (HPLC) over time in the 3-HP production process, and the result was shown in Table 2 and FIG. 2 below. Table 2 is the result of measuring the concentration (g/L) of 3-HP, glycerol, acetate and lactate according to the fermentation time after inoculation in the culture step for producing 3-HP (two-step culture).

[Table 2]

|  | 3-HP | Glycerol | Acetate | Lactate |
|---|---|---|---|---|
| Concentration before inoculation (g/L) | 0 | 71.68 | 0.47 | 0.31 |
| Concentration after fermentation for 29 hours (g/L) | 53.34 | 15.93 | 0 | 0 |
| 3-HP yield (%) | 95.7 | - | - | - |
| 3-HP productivity (g/L/h) | 2.94 | - | - | - |

As confirmed in FIG. 2, the 3-HP concentration was shown as 50.0g/L or more over 17 hours after inoculating the strain into the medium for producing 3-HP, and the productivity was confirmed as 2.94g/L/h, and thus the highest 3-HP productivity among values reported in documents to date was confirmed.

When 29 hours elapsed after inoculation, the final 3-HP concentration was confirmed as 53.3g/L, and the yield was about 95.7%, and the very excellent yield and 3-HP productivity were shown, while lactate and acetate, by-products of 3-HP production were hardly produced by 29 hours elapsed.

**Comparative example 1. Comparison to 1-step 3-HP production method**

To compare the 3-HP productivity of the two-step production method using a medium for producing 3-HP without glucose provided in the present application to the conventional one-step production method, the 3-HP producing strain prepared in Example 1-1 was cultured by a fed-batch culture method, and the cell growth and 3-HP production were simultaneously progressed by adding glucose required for cell growth and glycerol as a substrate to the culture medium (one-step production method).

The one-step production process performed for the comparison was specifically described as follows: the culture medium was used by adding glucose 20g/L and antibiotics for selection (streptomycin) 25mg/L to the MR medium of Example 1-2, and the temperature of 35 degrees Celsius was maintained. The pH was maintained as 6.95 using ammonia water, and the amount of dissolved oxygen (DO) was maintained as 20% by increasing the stirring rate stepwise by 900rpm, and the aeration was maintained as 1vvm.

When the glucose initially added in the culture medium was consumed, 3g/L of glucose was added in a continuous feeding method, and glycerol was added at 70g/L after 20 hours of culture. The subsequent process was performed in the same manner as Example 2.

The yield and productivity of 3-HP prepared by the 3-HP production process were measured by high pressure liquid chromatography (HPLC). The result of the 3-HP yield and productivity obtained in this way was shown in Table 3 below by comparing to the 3-HP yield and productivity by the two-step 3-HP production process conducted in Examples 1 and 2.

[Table 3]

|  | 1-step production method | 2-step production method |
|---|---|---|
| 3-HP yield (%) | 85 | 95.7 |
| 3-HP productivity (g/L/h) | 1.5 | 2.94 |
| By-products | Acetate 3 g/L<br>Lactate 2 g/L | - |

As confirmed in Table 3 above, the two-step 3-HP production method had the excellent 3-HP yield and productivity, compared to the conventional one-step production method. In addition, the excellence of the two-step production method of 3-HP was confirmed as the by-products, acetate and lactate were not produced in the two-step 3-HP production method, different from the one-step production method.


**Claims**

1. A method for manufacturing of 3-hydroxypropionic acid (3-HP), comprising:

    (1) performing high concentration cell culture of a 3-hydroxypropionic acid (3-HP) producing strain; and
    (2) producing 3-hydroxypropionic acid by separating the high concentration cells cultured and inoculating the high concentration cells separated to a medium for producing 3-hydroxypropionic acid,

    wherein in the step (2), cell proliferation does not occur.

2. The method according to claim 1, wherein the step (1) is performed by a fed-batch culture method, and the step (2) is performed by fermentation.

3. The method according to claim 1, wherein the step (1) is performed in a medium comprising glucose as a carbon source.

4. The method according to claim 1, wherein the step (1) is performed in a medium not comprising glycerol as a carbon source.

5.  The method according to claim 1, wherein the step (2) is performed in a medium comprising glycerol as a carbon source.

6. The method according to claim 1, wherein the step (2) is performed in a medium not comprising glucose as a carbon source.

7. The method according to claim 1, wherein the 3-HP producing strain comprises a gene encoding one or more proteins selected from the group consisting of glycerol dehydratase and aldehyde dehydrogenase.

8. The method according to any one of claim 1 to claim 7, wherein 3-hydroxypropionic acid yield is 80% or higher.

9. The method according to any one of claim 1 to claim 7, wherein 3-hydroxypropionic acid productivity is 2 g/L/h or more.

10. The method according to any one of claim 1 to claim 7, wherein the 3-hydroxypropionic acid is produced in 41g/L or more upon performing the step (2) for 29 hours.

11.  A culture solution of a 3-hydroxypropionic acid producing strain comprising 3-hydroxypropionic acid at a concentration of 45g/L or more.

12. The culture solution according to claim 11, wherein a concentration of a by-product is 0.1%(w/v) or less and the by-product is selected from the group consisting of acetate and lactate.

13. A composition for manufacturing 3-hydroxypropionic acid comprising the culture solution of claim 11 or claim 12.

【DRAWINGS】

【FIG. 1】

【FIG. 2】

## INTERNATIONAL SEARCH REPORT

| International application No. |
| --- |
| **PCT/KR2021/009988** |

**A. CLASSIFICATION OF SUBJECT MATTER**

**C12P 7/52**(2006.01)i; **C12N 15/70**(2006.01)i; **C12N 9/04**(2006.01)i; **C12N 9/02**(2006.01)i

According to International Patent Classification (IPC) or to both national classification and IPC

**B. FIELDS SEARCHED**

Minimum documentation searched (classification system followed by classification symbols)

C12P 7/52(2006.01); C12N 15/52(2006.01); C12N 15/63(2006.01); C12P 7/42(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 3-하이드록시프로피온산 (3-hydroxypropionic acid), 탄소원 (carbon source), 배지 (medium), 포도당 (glucose), 글리세롤 (glycerol), 발효배양 (fermentation), 유가배양 (fed-batch culture)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | KWAK, S. et al. Biosynthesis of 3-hydroxypropionic acid from glycerol in recombinant Escherichia coli expressing Lactobacillus brevis dhaB and dhaR gene clusters and E. coli K-12 aldH. Bioresource technology. 2013, vol. 135, pp. 432-439.<br>See abstract; and pages 433 and 438. | 11-13<br>1-10 |
| Y | KR 10-2010-0102928 A (KOREA RESEARCH INSTITUTE OF BIOSCIENCE AND BIOTECHNOLOGY) 27 September 2010 (2010-09-27)<br>See abstract; paragraphs [0028] and [0082]; and claim 1. | 1-10 |
| A | US 2014-0065681 A1 (JESSEN, H. et al.) 06 March 2014 (2014-03-06)<br>See abstract; paragraphs [0009] and [0141]; and claims 1 and 16-20. | 1-13 |
| A | KIM, M.-D. et al. Two-step fed-batch culture of recombinant Escherichia coli for production of Bacillus licheniformis maltogenic amylase. Journal of microbiology and biotechnology. 2002, vol. 12, no. 2, pp. 273-278.<br>See entire document. | 1-13 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **01 November 2021** | **01 November 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2021/009988** |

**C.     DOCUMENTS CONSIDERED TO BE RELEVANT**

| Category* | Citation of document, with indication, where appropriate, of the relevant passages | Relevant to claim No. |
| --- | --- | --- |
| A | MATSAKAS, L. et al. Biological production of 3-hydroxypropionic acid: an update on the current status. Fermentation. 2018, vol. 4, thesis no. 13, pp. 1-21.<br>     See entire document. | 1-13 |

## INTERNATIONAL SEARCH REPORT
### Information on patent family members

| International application No. |
|---|
| **PCT/KR2021/009988** |

| Patent document cited in search report | | | Publication date (day/month/year) | Patent family member(s) | | | Publication date (day/month/year) |
|---|---|---|---|---|---|---|---|
| KR | 10-2010-0102928 | A | 27 September 2010 | KR | 10-1189187 | B1 | 10 October 2012 |
| US | 2014-0065681 | A1 | 06 March 2014 | AU | 2011-336923 | A1 | 30 May 2013 |
| | | | | AU | 2011-336923 | B2 | 16 February 2017 |
| | | | | BR | 112013012630 | A2 | 24 September 2020 |
| | | | | CA | 2818499 | A1 | 07 June 2012 |
| | | | | CN | 103502432 | A | 08 January 2014 |
| | | | | CN | 107828671 | A | 23 March 2018 |
| | | | | EP | 2643450 | A2 | 02 October 2013 |
| | | | | EP | 3287519 | A1 | 28 February 2018 |
| | | | | EP | 3287519 | B1 | 18 March 2020 |
| | | | | JP | 2013-542747 | A | 28 November 2013 |
| | | | | JP | 6061862 | B2 | 18 January 2017 |
| | | | | KR | 10-2013-0119945 | A | 01 November 2013 |
| | | | | MX | 2013005654 | A | 17 July 2013 |
| | | | | US | 10260072 | B2 | 16 April 2019 |
| | | | | US | 10633664 | B2 | 28 April 2020 |
| | | | | US | 11118187 | B2 | 14 September 2021 |
| | | | | US | 2012-0135481 | A1 | 31 May 2012 |
| | | | | US | 2016-0177317 | A1 | 23 June 2016 |
| | | | | US | 2018-0044684 | A1 | 15 February 2018 |
| | | | | US | 2019-0249181 | A1 | 15 August 2019 |
| | | | | US | 2020-0291408 | A1 | 17 September 2020 |
| | | | | US | 9090918 | B2 | 28 July 2015 |
| | | | | US | 9777280 | B2 | 03 October 2017 |
| | | | | WO | 2012-074818 | A2 | 07 June 2012 |
| | | | | WO | 2012-074818 | A3 | 27 September 2012 |
| | | | | ZA | 201303544 | B | 26 November 2014 |

Form PCT/ISA/210 (patent family annex) (July 2019)

**REFERENCES CITED IN THE DESCRIPTION**

**Patent documents cited in the description**

• KR 1020200096238